# EUROPÄISCHE PATENTSCHRIFT

(11) **EP 3 362 653 B1**
(45) Veröffentlichungstag und Bekanntmachung des Hinweises auf die Patenterteilung: **23.01.2019**
(21) Anmeldenummer: 17707247.7
(22) Anmeldetag: 23.02.2017
(51) Int. Cl.: F01N 1/06, B60Q 5/00

(54) **KRAFTFAHRZEUG MIT EINER STEUERVORRICHTUNG FÜR ZUMINDEST EINEN LAUTSPRECHER EINER ABGASANLAGE DES KRAFTFAHRZEUGS**
MOTOR VEHICLE HAVING A CONTROL APPARATUS FOR AT LEAST ONE LOUDSPEAKER OF AN EXHAUST SYSTEM OF THE MOTOR VEHICLE
VÉHICULE ÉQUIPÉ D'UN DISPOSITIF DE COMMANDE D'AU MOINS UN HAUT-PARLEUR D'UN SYSTÈME D'ÉCHAPPEMENT DU VÉHICULE AUTOMOBILE

(30) Priorität: 27.02.2016 DE 102016002449
(43) Veröffentlichungstag der Anmeldung: 22.08.2018
(73) Patentinhaber: AUDI AG, 85045 Ingolstadt (DE)
(72) Erfinder: RÜEGG, Markus, 71292 Friolzheim (DE)
(86) Internationale Anmeldenummer: PCT/EP2017/054178
(87) Internationale Veröffentlichungsnummer: WO 2017/144592

(56) Entgegenhaltungen:
- EP-A2- 1 193 683
- WO-A1-00/05489
- WO-A1-2016/026889
- WO-A1-2016/026890
- DE-A1-102011 116 635

## Beschreibung

Die Erfindung betrifft ein Kraftfahrzeug, das eine sogenannte aktive Abgasanlage (aktive AGA) aufweist. Mit anderen Worten ist in die Abgasanlage zumindest ein Lautsprecher integriert, welcher jeweils einen Schall in einem jeweiligen Rohr der Abgasanlage abstrahlen kann, um hierdurch beispielsweise ein Motorgeräusch eines Verbrennungsmotors des Kraftfahrzeugs zu kompensieren und/oder durch ein vorbestimmtes anderes Motorgeräusch zumindest teilweise zu ersetzen. Zu der Erfindung gehört auch ein entsprechendes Verfahren zum Steuern oder Betreiben des zumindest einen Lautsprechers.

Eine Abgasanlage mit integrierten Lautsprechern ist aus der DE 10 2013 112 409 A1 bekannt. Darin sind Mikrofone dazu vorgesehen, ein aktuelles Motorgeräusch eines Verbrennungsmotors des Kraftfahrzeugs zu erfassen. Mittels der Mikrofonsignale wird ein Gegenschallsignal erzeugt, das mittels der in die Abgasanlage integrierten Lautsprecher in ein Endrohr der Abgasanlage abgestrahlt wird, sodass das Motorgeräusch und der Gegenschall sich kompensieren. In einer Umgebung des Kraftfahrzeugs sind somit die Motorgeräusche weniger deutlich hörbar.

Aus der DE 10 2013 221 182 A1 ist bekannt, von einem Kraftfahrzeug aus in eine Fahrzeugumgebung akustische Klangsignale abzustrahlen, beispielsweise ein Piep- oder Klicksignal, um hierdurch eine Warnung oder einen Hinweis an eine außerhalb des Kraftfahrzeugs befindliche Person auszugeben. Das Kraftfahrzeug benötigt hierzu dedizierte Lautsprecher, welche das Kraftfahrzeug in der Herstellung teurer machen.

In der DE 10 2011 051 284 A1 ist ein Soundmodul für ein Kraftfahrzeug beschrieben, das sich mit kurzem Signalweg an einen Kommunikationsbus des Kraftfahrzeugs anschließen lässt. Allerdings ist dieses Soundmodul nur für den Einsatz im Fahrzeuginnenraum geeignet. Ähnliche Systeme sind aus der WO2016/026889A1 oder der DE102011116635A1 bekannt.

Der Erfindung liegt die Aufgabe zugrunde, von einem Kraftfahrzeug aus ein Hinweissignal in eine Fahrzeugumgebung abzugeben.

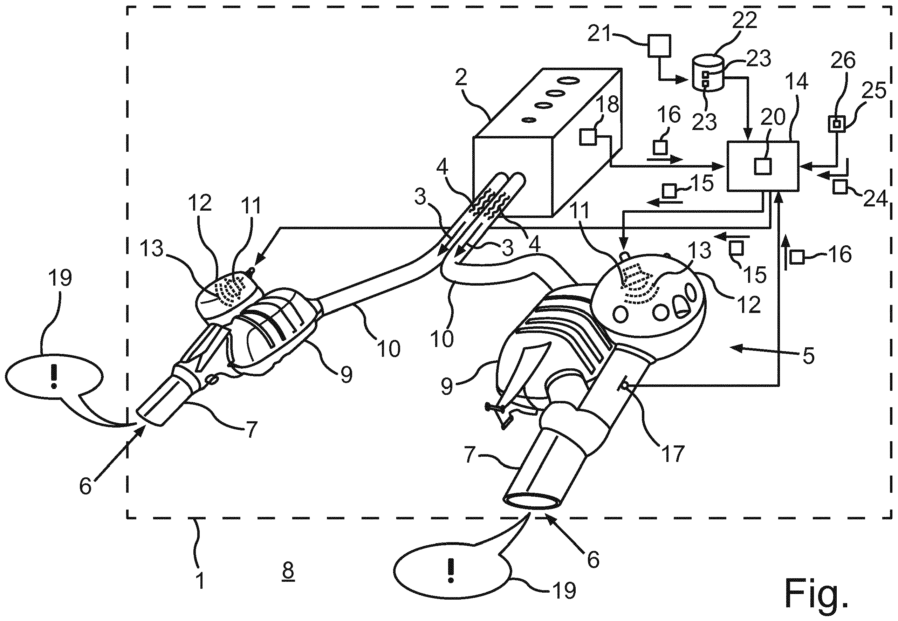

Die Aufgabe wird durch den Gegenstand des unabhängigen Patentanspruchs 1 gelöst. Vorteilhafte Weiterbildungen der Erfindung ergeben sich durch die Merkmale der abhängigen Patentansprüche, die folgende Beschreibung sowie die Figur.

Durch die Erfindung ist ein Kraftfahrzeug bereitgestellt, dass eine sogenannte aktive Abgasanlage aufweist. Das Kraftfahrzeug weist somit einen Verbrennungsmotor auf, welcher im Betrieb ein Verbrennungsgas erzeugt, sowie eine Abgasanlage zum Abführen des Verbrennungsgases von dem Verbrennungsmotor zu zumindest einer Auslassöffnung. Eine solche Auslassöffnung kann beispielsweise in einem jeweiligen Endrohr der Abgasanlage bereitgestellt sein. Eine Erfassungseinrichtung ist dazu eingerichtet, ein Zustandssignal zu erfassen, welches im Betrieb des Verbrennungsmotors ein aktuelles Motorgeräusch desselben und/oder einen aktuellen, den Betrieb charakterisierenden Betriebsparameter beschreibt. Die Erfassungseinrichtung kann also beispielsweise ein Mikrofon umfassen, welches das aktuelle Motorgeräusch erfasst. Die Erfassungseinrichtung kann aber auch beispielsweise zum Erfassen einer aktuellen Drehzahl des Verbrennungsmotors eingerichtet sein, um hierdurch als Betriebsparameter die Drehzahl zu erfassen. Des Weiteren ist zumindest ein Lautsprecher bereitgestellt, welcher dazu eingerichtet ist, einen Schall in ein jeweiliges Rohr der Abgasanlage zu der zumindest einen Auslassöffnung hin abzustrahlen. Der zumindest eine Lautsprecher erzeugt den Schall also in der Abgasanlage, sodass der Schall aus der Abgasanlage heraus durch die zumindest eine Auslassöffnung nach außen dringt. Eine Steuervorrichtung ist dazu eingerichtet, den zumindest einen Lautsprecher zum Erzeugen des Schalls in Abhängigkeit von dem Zustandssignal der Erfassungseinrichtung anzusteuern und hierdurch mittels des erzeugten Schalls das Motorgeräusch zumindest teilweise zu kompensieren und/oder durch ein vorbestimmtes anderes Motorgeräusch zumindest teilweise zu ersetzen. Es wird also entweder das Motorgeräusch in der Fahrzeugumgebung reduziert und/oder ein anderes Motorgeräusch, beispielsweise das eines Verbrennungsmotors eines anderen Motortyps, wird ausgegeben oder abgestrahlt.

Um nun in der Fahrzeugumgebung auch ein Hinweissignal an eine Person auszugeben, ist erfindungsgemäß vorgesehen, dass die Steuervorrichtung dazu eingerichtet ist, den zumindest einen Lautsprecher zusätzlich unabhängig von dem Zustandssignals, also unabhängig vom aktuellen Motorgeräusch und/oder Betriebsparameter des Verbrennungsmotors, auf der Grundlage von vorbestimmten, gespeicherten Audiodaten anzusteuern und hierdurch mittels des zumindest einen Lautsprechers ein durch die Audiodaten beschriebenes Hinweissignal akustisch auszugeben. Die Lautsprecher erzeugen entsprechend einen Schall, welcher das Hinweissignal repräsentiert oder trägt. Der Schall dringt durch die Abgasanlage und deren zumindest eine Auslassöffnung in die Fahrzeugumgebung abgestrahlt, sodass das Hinweissignal außerhalb des Kraftfahrzeugs hörbar wird. Hierbei wird in vorteilhafter Weise kein zusätzlicher Lautsprecher benötigt, da der zumindest eine Lautsprecher der aktiven Abgasanlage zum Erzeugen des akustischen Hinweissignals genutzt werden kann.

Durch den Betrieb der Steuervorrichtung des erfindungsgemäßen Kraftfahrzeugs ergibt sich ein Verfahren, das ebenfalls Bestandteil der Erfindung ist. Bei dem Verfahren zum Betreiben der Steuervorrichtung für den zumindest einen Lautsprecher der Abgasanlage ist vorgesehen, dass die Steuervorrichtung den zumindest einen Lautsprecher zum Erzeugen eines Schalls in Abhängigkeit von dem beschriebenen Zustandssignal ansteuert und hierdurch mittels des von dem zumindest einen Lautsprecher erzeugten Schalls das durch das Zustandssignal beschriebene Motorgeräusch zumindest teilweise kompensiert und/oder durch ein vorbestimmtes anderes Motorgeräusch zumindest teilweise ersetzt. Zusätzlich ist erfindungsgemäß vorgesehen, dass die Steuervorrichtung den zumindest einen Lautsprecher gleichzeitig und/oder zu einem anderen Zeitpunkt unabhängig von dem Zustandssignal, auf der Grundlage von vorbestimmten gespeicherten Audiodaten ansteuert und hierdurch mittels des zumindest einen Lautsprechers ein durch die Audiodaten beschriebenes Hinweissignal ausgibt.

Zu der Erfindung gehören optionale Weiterbildungen, durch deren Merkmale sich zusätzliche Vorteile ergeben.

Da das Hinweissignal unabhängig von dem Zustandssignal erzeugt wird, ist es somit auch unabhängig von einem Betrieb des Verbrennungsmotors. Entsprechend sieht eine Weiterbildung vor, dass die Steuervorrichtung dazu eingerichtet ist, das Hinweissignal bei abgestelltem oder ausgeschaltetem Verbrennungsmotor auszugeben. Der Verbrennungsmotor erzeugt kein Motorgeräusch. Die Zündung des Kraftfahrzeugs kann abgeschaltet sein. Hierdurch ergibt sich der Vorteil, dass ein solches Hinweissignal erzeugt werden kann, dass ein Benutzer des Kraftfahrzeugs erhalten kann, während er das Kraftfahrzeug von außerhalb desselben bedient, beispielsweise das Kraftfahrzeug abschließt.

Erfindungsgemäß wird das Hinweissignal gezielt in Abhängigkeit von zumindest einem vorbestimmten Ereignis erzeugt. Die Erfindung sieht hierzu vor, dass eine Detektionseinrichtung bereitgestellt und dazu eingerichtet ist, zumindest eine vorbestimmte Ausgabesituation zu detektieren und durch ein von dem Zustandssignal unabhängiges Auslösesignal diese Ausgabesituation jeweils zu signalisieren. Die Steuervorrichtung ist dazu eingerichtet, in Abhängigkeit von dem Auslösesignal das Hinweissignal auszugeben. Eine solche Ausgabesituation oder ein Ausgabeereignis kann jeweils durch eine Detektionseinrichtung detektiert werden, die entsprechend der zu detektierenden Ausgabesituation ausgestaltet werden kann. Beispielsweise kann als eine mögliche Detektionseinrichtung eine Alarmanlage das Auslösesignal bei einem Einbruchsversuch erzeugen. Durch die Weiterbildung ergibt sich der Vorteil, dass das Hinweissignal als eine akustische Rückmeldung zu der detektierten Ausgabesituation erzeugt wird und hierdurch ein Benutzer des Kraftfahrzeugs oder auch eine sich in der Fahrzeugumgebung befindliche Person informiert oder gewarnt werden kann.

Eine Weiterbildung hierzu sieht vor, dass die Detektionseinrichtung dazu eingerichtet ist, als Ausgabesituation eine Parkphase des Kraftfahrzeugs zu detektieren, in welcher das Kraftfahrzeug geparkt oder abgestellt und zumindest der Fahrer des Kraftfahrzeugs aus dem Kraftfahrzeug ausgestiegen ist. Durch das Hinweissignal kann somit dem ausgestiegenen Fahrer z.B. signalisiert werden, dass das Kraftfahrzeug für die Parkphase bereit oder eingestellt ist. Beispielsweise kann also signalisiert sein, dass kein elektrischer Verbraucher mehr unnötig betrieben wird, wie beispielsweise das Infotainmentsystem (Informations-Unterhaltungssystem) und/oder die Fahrzeugleuchte, und/oder dass die Alarmanlage aktiviert worden ist. Es kann hierdurch auch signalisiert werden, dass noch Handlungsbedarf besteht, weil beispielsweise ein Fenster des Kraftfahrzeugs und/oder ein Schiebedach geöffnet sind. Entsprechende Detektionseinrichtungen zum Detektieren der beschriebenen Zustände sind an sich mit Mitteln aus dem Stand der Technik bereitstellbar.

Erfindungsgemäß ist die Detektionseinrichtung dazu eingerichtet, als Ausgabesituation einen Zustandswechsel einer Schließanlage für Fahrzeugtüren des Kraftfahrzeugs zu detektieren. Mit anderen Worten wird das Hinweissignal beim Abschließen und/oder beim Aufschließen des Kraftfahrzeugs erzeugt. Hierdurch ergibt sich der Vorteil, dass einem Benutzer des Kraftfahrzeugs die erfolgreiche Betätigung der Schließanlage signalisiert wird. Zudem kann ein Kraftfahrzeug bei Benutzung eines Funkschlüssels durch das Hinweissignal darauf aufmerksam machen, wo es sich befindet.

Erfindungsgemäß ist die Detektionseinrichtung dazu eingerichtet, nach Detektieren einer Ausgabesituation das zugehörige Auslösesignal mit einer vorbestimmten Zeitverzögerung zu erzeugen. Die Zeitverzögerung kann voreingestellt sein oder beispielsweise durch einen Zufallsgenerator erzeugt werden. Durch Vorsehen einer Zeitverzögerung ergibt sich der Vorteil, dass einem Benutzer des Kraftfahrzeugs Zeit gegeben wird, sich in Position zu bringen, um das Hinweissignal nutzen zu können. Beispielsweise kann in dieser Weise nach dem Entriegeln des Kraftfahrzeugs durch einen Funkschlüssel erst eine Annäherung eines Benutzers abgewartet werden, ohne dass eine aufwendige Detektion einer Position des Benutzers nötig ist. Man geht einfach davon aus, dass der Benutzer erst nach der Zeitverzögerung neben dem Kraftfahrzeug steht.

Eine Weiterbildung sieht vor, dass die Detektionseinrichtung dazu eingerichtet ist, als Ausgabesituation eine vorbestimmte Betätigungshandlung eines Benutzers an einer Bedieneinrichtung für das Kraftfahrzeug zu detektieren. Beispielsweise kann detektiert werden, dass ein Benutzer eine Schließtaste an einem Funkschlüssel gemäß einem vorbestimmten Bedienmuster betätigt, beispielsweise zweimal hintereinander drückt. Hierdurch ergibt sich der Vorteil, dass das Hinweissignal gezielt durch den Benutzer ausgelöst oder gesteuert werden kann. So kann der Benutzer beispielsweise durch die Betätigungshandlung das Ausgeben des Hinweissignals bezüglich der geöffneten Fenster und/oder noch in Betrieb befindlicher elektrischer Verbraucher ausgeben lassen und hierdurch überprüfen, ob das Kraftfahrzeug parkfertig oder parkbereit ist.

Eine Ausführungsform sieht vor, dass die Detektionseinrichtung dazu eingerichtet ist, als Ausgabesituation einen bei laufendem Verbrennungsmotor eingelegten Rückwärtsgang zu detektieren. Hierdurch ergibt sich der Vorteil, dass mittels des zumindest einen Lautsprechers als das Hinweissignal eine Warnung vor einer möglichen Rückwärtsfahrt des Kraftfahrzeugs in die Fahrzeugumgebung ausgegeben wird. Das Hinweissignal kann dann beispielsweise ein Rattern oder ein Rauschen oder ein Ton sein, um die Aufmerksamkeit einer Person in der Fahrzeugumgebung auf das Kraftfahrzeug zu lenken.

Eine Weiterbildung sieht vor, dass die Steuervorrichtung dazu eingerichtet ist, das Hinweissignal nur zu erzeugen, falls zumindest ein vorbestimmter Fahrmodus aus mehreren aktivierbaren Fahrmodi des Kraftfahrzeugs in dem Kraftfahrzeug aktiviert ist. Ein Fahrmodus zeichnet sich dadurch aus, dass er eine vorbestimmte Betriebsweise des Verbrennungsmotors und/oder eines Fahrwerks des Kraftfahrzeugs festlegt. Jeder Fahrmodus stellt hierbei eine andere Betriebsweise ein. Somit kann die Ausgabe des Hinweissignals beispielsweise auf einen Sportfahrmodus begrenzt oder beschränkt werden.

Natürlich ist gemäß einer Weiterbildung auch vorgesehen, dass ein Benutzer des Kraftfahrzeugs mittels einer Betätigungs- oder Bedieneinrichtung das Ausgeben des Hinweissignals aktivieren und wahlweise deaktivieren kann.

Eine Weiterbildung sieht vor, dass die Steuervorrichtung dazu eingerichtet ist, als das Hinweissignal ein Klopfen, insbesondere ein imitiertes Herzklopfen, auszugeben. Ein Klopfen als Hinweissignal weist den Vorteil auf, dass es auch dann noch als solches in der Fahrzeugumgebung wahrnehmbar ist, wenn das zumindest eine Rohr, in welches der Schall durch den zumindest einen Lautsprecher abgestrahlt wird, eine akustische Übertragungscharakteristik aufweist, die einen frequenzabhängigen Betragsgang aufweist, welcher Frequenzen des Schalls unterschiedlich dämpft. Ein Klopfen zeichnet sich nicht primär durch das Spektrum seiner Frequenzen aus, sondern durch seine zeitliche Modulation. Diese wird aber durch das weitestgehend lineare Übertragungsverhalten eines Rohres nicht beeinträchtigt.

Natürlich kann auch vorgesehen sein, als Hinweissignal eine Melodie und/oder ein Geräusch auszugeben. Dieses wird dann bevorzugt dahingehend angepasst, dass es die akustische Übertragungscharakteristik der Abgasanlage berücksichtigt, sodass in der Fahrzeugumgebung nach dem Austreten des Schalls aus der zumindest einen Auslassöffnung ein gewünschtes Zielgeräusch hörbar oder wahrnehmbar oder messbar ist.

Eine Weiterbildung sieht vor, dass eine Konfigurationseinrichtung dazu eingerichtet ist, die Audiodaten in Abhängigkeit von einer Benutzereingabe einzustellen und/oder aus mehreren vorbestimmten Audiodatensätzen auszuwählen. Mit anderen Worten kann ein Benutzer einstellen, welches Hinweissignal ausgegeben werden soll. Beispielsweise kann also als jeweiliger Audiodatensatz eine MP3-Datei vorgegeben sein. Der Benutzer kann dann mittels der Konfigurationseinrichtung, die beispielsweise durch das besagte Infotainmentsystem realisiert sein kann, diejenigen Audiodaten auswählen, die durch die Steuervorrichtung mittels des zumindest einen Lautsprechers wiedergegeben werden sollen.

Das erfindungsgemäße Kraftfahrzeug ist bevorzugt als Kraftwagen, insbesondere als Personenkraftwagen oder Lastkraftwagen, ausgestaltet.

Zu der Erfindung gehören auch Weiterbildungen des erfindungsgemäßen Verfahrens, die Merkmale aufweisen, wie sie bereits im Zusammenhang mit den Weiterbildungen des erfindungsgemäßen Kraftfahrzeugs beschrieben worden sind. Aus diesem Grund sind die entsprechenden Weiterbildungen des erfindungsgemäßen Verfahrens hier nicht noch einmal beschrieben.

Im Folgenden ist ein Ausführungsbeispiel der Erfindung beschrieben. Hierzu zeigt die einzige Figur (Fig.) eine schematische Darstellung einer Ausführungsform des erfindungsgemäßen Kraftfahrzeugs.

Bei dem im Folgenden erläuterten Ausführungsbeispiel handelt es sich um eine bevorzugte Ausführungsform der Erfindung. Bei dem Ausführungsbeispiel stellen die beschriebenen Komponenten der Ausführungsform jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren ist die beschriebene Ausführungsform auch durch weitere der bereits beschriebenen Merkmale der Erfindung ergänzbar.

Die Figur zeigt ein Kraftfahrzeug 1, bei dem es sich beispielsweise um einen Kraftwagen, insbesondere einen Personenkraftwagen, handeln kann. Das Kraftfahrzeug 1 weist einen Verbrennungsmotor 2 auf, bei dem es sich beispielsweise um einen Dieselmotor oder einen Ottomotor handeln kann. Im Betrieb oder eingeschalteten Zustand erzeugt der Verbrennungsmotor 2 ein Verbrennungsgas 3 und Motorgeräusche 4, die durch eine Abgasanlage 5 des Kraftfahrzeugs 1 in an sich bekannter Weise zu zumindest einer Auslassöffnung 6 der Abgasanlage 5 geführt oder geleitet werden. Die zumindest eine Auslassöffnung 6 kann beispielsweise durch ein jeweiliges Endrohr 7 der Abgasanlage 5 gebildet sein. Durch die Auslassöffnung 6 kann das Verbrennungsgas 3 und das Motorgeräusch 4 in eine Fahrzeugumgebung 8 entweichen. Von der Abgasanlage 5 sind zur Veranschaulichung des Weiteren Schalldämpfer 9 und die Schalldämpfer 9 mit dem Verbrennungsmotor 2 koppelnde oder verbindende Rohre 10 dargestellt. Der Verbrennungsmotor 2 ist somit über zumindest ein Rohr 10 und zumindest einen Schalldämpfer 9 mit der jeweiligen Auslassöffnung 6, beispielsweise in einem Endrohr 7, gekoppelt oder verbunden.

Bei dem Kraftfahrzeug 1 weist die Abgasanlage 5 zusätzlich zumindest einen Lautsprecher 11 auf, der beispielsweise jeweils in einem Lautsprechertopf 12 angeordnet sein kann. Jeder Lautsprecher 11 kann einen Schall 13 erzeugen, der von dem Lautsprecher 11 in der Abgasanlage 5 beispielsweise zu dem Endrohr 7 zur Auslassöffnung 6 hin abgestrahlt wird oder dorthin gelangt. Der Lautsprechertopf 12 kann hierbei den Schall 13 zu der Auslassöffnung 6 hin reflektieren oder führen oder bündeln.

Der zumindest eine Lautsprecher 11 kann durch eine Steuervorrichtung 14 gesteuert oder betrieben werden. Die Steuervorrichtung 14 kann hierzu beispielsweise auf der Grundlage eines Steuergeräts des Kraftfahrzeugs 1 realisiert sein. Zum Steuern des zumindest einen Lautsprechers 11 erzeugt die Steuervorrichtung 14 ein jeweiliges Lautsprechersignal 15. Das Lautsprechersignal 15 erzeugt die Steuervorrichtung 14 dabei in Abhängigkeit von zumindest einem Zustandssignal 16, durch welches das Motorgeräusch 4 beschrieben ist. Beispielsweise kann ein Zustandssignal 16 mittels eines Mikrofons 17 erzeugt werden, welches das Motorgeräusch 4 in der Abgasanlage 5 erfasst. Durch eine Drehzahlerfassungseinrichtung 18 kann als Zustandssignal 16 beispielsweise eine aktuelle Drehzahl des Verbrennungsmotors 2 ermittelt und signalisiert werden. Die Steuervorrichtung 14 kann auf der Grundlage des zumindest einen Zustandssignals 16 das Lautsprechersignal 15 derart in an sich bekannter Weise erzeugen, dass an der zumindest einen Auslassöffnung 6 das Motorgeräusch 4 durch destruktive Interferenz reduziert wird. Zusätzlich oder alternativ dazu kann vorgesehen sein, dass mittels des Lautsprechersignals 15 als Schall 13 ein Motorgeräusch erzeugt wird, das sich von dem Motorgeräusch 4 unterscheidet, sodass in der Fahrzeugumgebung 8 ein Motorgeräusch eines anderen Motortyps zu hören ist.

Bei dem Kraftfahrzeug 1 wird der zumindest eine Lautsprecher 11 zusätzlich dazu verwendet oder betrieben, in der Fahrzeugumgebung 8 ein akustisches Hinweissignal 19 zu erzeugen oder abzustrahlen. Dieses Hinweissignal 19 ist vom Klang und Ausgabezeitpunkt unabhängig vom Betrieb des Verbrennungsmotors 2. Das Hinweissignal 19 wird mittels des zumindest einen Lautsprechers 11 abgestrahlt. Das entsprechende Lautsprechersignal 15 kann auf der Grundlage von Audiodaten 20 erzeugt werden, die beispielsweise als das Hinweissignal 19 eine Melodie und/oder eine Ansage und/oder ein Klopfen und/oder ein Geräusch beschreiben. Es kann vorgesehen sein, dass ein Benutzer mittels einer Konfigurationseinrichtung 21 aus einem Datenspeicher 22 einen dort gespeicherten Audiodatensatz 23 auswählt und den ausgewählten Audiodatensatz als die Audiodaten 20 in der Steuervorrichtung 14 festlegt. Die Steuervorrichtung 14 kann die Audiodaten 20 immer dann als das akustische Hinweissignal 19 mittels des zumindest einen Lautsprechers 11 in der Fahrzeugumgebung 8 erzeugen, falls sie ein vorbestimmtes Auslösesignal 24 empfängt. Das Auslösesignal 24 kann durch eine Detektionseinrichtung 25 erzeugt werden, die das Auslösesignal 24 immer dann erzeugt, wenn sie eine vorbestimmte Ausgabesituation 26 detektiert. Beispielsweise kann die Auslösesituation 26 umfassen, dass das Kraftfahrzeug 1 abgestellt wurde und/oder abgeschlossen wurde.

Ist also bei dem Kraftfahrzeug 1 eine aktive Abgasanlage 5 verbaut, um einen Motorklang eines großvolumigeren Verbrennungsmotors als dem Verbrennungsmotor 2 zu simulieren, so kann mittels der Steuervorrichtung 14 unter Nutzung des zumindest einen Lautsprechers 11 der aktiven Abgasanlage 5 auch ein akustisches Markenzeichen oder Hinweissignal 19 in einer vorbestimmten Ausgabesituation 26 über den zumindest einen Lautsprecher 11 wiedergegeben werden. Somit ist beispielsweise ein Markenjingle oder ein charakteristisches akustisches Hinweissignal 19 außerhalb des Kraftfahrzeugs 1 in der angrenzenden Fahrzeugumgebung 8 hörbar. So kann beispielsweise ein Herzklopfen als Hinweissignal 19 ausgegeben werden, um hierdurch beispielsweise ein Abschiedssignal oder ein Willkommenssignal für einen Benutzer des Kraftfahrzeugs 1 in der Fahrzeugumgebung 8 bereitzustellen.

Insgesamt zeigt das Beispiel, wie durch die Erfindung markenbezogene Jingles mit einer aktiven Abgasanlage (AGA) wiedergeben werden können.

## Patentansprüche

1. Kraftfahrzeug (1), aufweisend:
- einen Verbrennungsmotor (2), welcher im Betrieb ein Verbrennungsgas (3) erzeugt,
- eine Abgasanlage (5) zum Abführen des Verbrennungsgases (5) von dem Verbrennungsmotor (2) zu zumindest einer Auslassöffnung (6),
- eine Erfassungseinrichtung (17, 18) zum Erzeugen eines Zustandssignals (16), welches im Betrieb des Verbrennungsmotors (2) ein aktuelles Motorgeräusch (4) desselben und/oder einen aktuellen, den Betrieb charakterisierenden Betriebsparameter beschreibt,
- zumindest einen Lautsprecher (11), welcher dazu eingerichtet ist, einen Schall (13) in ein jeweiliges Rohr (7) der Abgasanlage (3) zu der zumindest einen Auslassöffnung (6) hin abzustrahlen, und
- eine Steuervorrichtung (14), welche dazu eingerichtet ist, den zumindest einen Lautsprecher (11) zum Erzeugen des Schalls (13) in Abhängigkeit von dem Zustandssignal (16) anzusteuern und hierdurch mittels des erzeugten Schalls (13) das Motorgeräusch (4) zumindest teilweise zu kompensieren und/oder durch ein vorbestimmtes anderes Motorgeräusch zumindest teilweise zu ersetzen,
**dadurch gekennzeichnet, dass**
die Steuervorrichtung (14) dazu eingerichtet ist, den zumindest einen Lautsprecher (11) zusätzlich unabhängig von dem Zustandssignal (16) auf der Grundlage von vorbestimmten, gespeicherten Audiodaten (20) anzusteuern und hierdurch mittels des zumindest einen Lautsprechers (11) ein durch die Audiodaten (20) beschriebenes Hinweissignal (19) akustisch auszugeben, wobei eine Detektionseinrichtung (25) bereitgestellt und dazu eingerichtet ist, eine vorbestimmte Ausgabesituation (26), welche zumindest einen Zustandswechsel einer Schließanlage für Fahrzeugtüren des Kraftfahrzeugs (1) umfasst, zu detektieren und durch ein von dem Zustandssignals (16) unabhängiges, in der Ausgabesituation mit einer vorbestimmten Zeitverzögerung erzeugtes Auslösesignal (24) zu signalisieren, und die Steuervorrichtung (14) dazu eingerichtet ist, in Abhängigkeit von dem Auslösesignal (24) das Hinweissignal (19) auszugeben.

2. Kraftfahrzeug (1) nach Anspruch 1, wobei die Steuervorrichtung (14) dazu eingerichtet ist, das Hinweissignal (19) bei abgestelltem Verbrennungsmotor (2) auszugeben.

3. Kraftfahrzeug (1) nach einem der vorhergehenden Ansprüche, wobei die Detektionseinrichtung (25) dazu eingerichtet ist, als Ausgabesituation (26) eine Parkphase zu detektieren, in welcher das Kraftfahrzeug (1) geparkt und ein Fahrer aus dem Kraftfahrzeug (1) ausgestiegen ist.

4. Kraftfahrzeug (1) nach einem der vorhergehenden Ansprüche, wobei die Detektionseinrichtung (25) dazu eingerichtet ist, als Ausgabesituation (26) eine vorbestimmte Betätigungshandlung eines Benutzers an einer Bedieneinrichtung für das Kraftfahrzeug (1) zu detektieren.

5. Kraftfahrzeug (1) nach einem der vorhergehenden Ansprüche, wobei die Detektionseinrichtung (25) dazu eingerichtet ist, als Ausgabesituation (26) einen bei laufendem Verbrennungsmotor (2) eingelegten Rückwärtsgang zu detektieren.

6. Kraftfahrzeug (1) nach einem der vorhergehenden Ansprüche, wobei die Steuervorrichtung (14) dazu eingerichtet ist, das Hinweissignal (19) nur zu erzeugen, falls zumindest ein vorbestimmter Fahrmodus aus mehreren aktivierbaren Fahrmodi, durch welche jeweils eine andere Betriebsweise des Verbrennungsmotors (2) und/oder eines Fahrwerks des Kraftfahrzeugs (1) festgelegt ist, in dem Kraftfahrzeug (1) aktiviert ist.

7. Kraftfahrzeug (1) nach einem der vorhergehenden Ansprüche, wobei die Steuervorrichtung (14) dazu eingerichtet ist, als das Hinweissignal (19) ein Klopfen, insbesondere ein imitiertes Herzklopfen, auszugeben.

8. Kraftfahrzeug (1) nach einem der vorhergehenden Ansprüche, wobei eine Konfigurationseinrichtung (21) dazu eingerichtet ist, die Audiodaten (20) in Abhängigkeit von einer Benutzereingabe einzustellen und/oder aus mehreren vorbestimmten Audiodatensätzen (23) auszuwählen.

## Claims

1. Motor vehicle (1), having:
- an internal combustion engine (2), which generates a combustion gas (3) during operation,
- an exhaust system (5) for taking away the combustion gas (5) of the internal combustion engine (2) to at least one outlet opening (6),
- a detection device (17, 18) for generating a state signal (16), which during operation of the internal combustion engine (2) describes a current engine noise (4) thereof and/or a current operating parameter characterising the operation,
- at least one loudspeaker (11), which is configured to radiate a sound (13) into a respective pipe (7) of the exhaust system (3) towards the at least one outlet opening (6), and
- a control device (14), which is configured to control the at least one loudspeaker (11) for generating the sound (13) in response to the state signal (16) and thereby, by means of the generated sound (13), to at least partially compensate the motor noise (4) and/or to at least partially replace same with another predetermined engine noise,
**characterised in that**
the control device (14) is configured to additionally control the at least one loudspeaker (11) independently of the state signal (16) on the basis of predetermined, stored audio data (20) and thereby, by means of the at least one loudspeaker (11) to output an acoustic advisory signal (19) described by the audio data (20), wherein a detection device (25) is provided and configured to detect a predetermined output situation (26), which comprises at least one state change of a closing system for vehicle doors of the motor vehicle (1), to detect and to signal, through a trigger signal (24) which is independent of the state signal (16) and generated in the output situation with a predetermined time delay, and the control device (14) is configured to output the advisory signal (19) depending on the trigger signal (24).

2. Motor vehicle (1) according to claim 1, wherein the control device (14) is configured to output the advisory signal (19) when the internal combustion engine (2) is switched off.

3. Motor vehicle (1) according to one of the preceding claims, wherein the detection device (25) is configured to detect a parking phase as output situation (26), in which the motor vehicle (1) is parked and a driver climbs out of the motor vehicle (1).

4. Motor vehicle (1) according to one of the preceding claims, wherein the detection device (25) is configured to detect as output situation (26) a predetermined actuating action of a user on an operating device for the motor vehicle (1).

5. Motor vehicle (1) according to one of the preceding claims, wherein the detection device (25) is configured to detect as output situation (26) a reverse gear engaged during running of the internal combustion engine (2).

6. Motor vehicle (1) according to one of the preceding claims, wherein the control device (14) is configured to generate the advisory signal (19) only in the event that at least one predetermined driving mode, from a plurality of activatable driving modes, through which another mode of operation of the internal combustion engine (2) and/or of a driving of the motor vehicles (1) is set respectively, is activated in the motor vehicle (1).

7. Motor vehicle (1) according to one of the preceding claims, wherein the control device (14) is configured to output a knocking as the advisory signal (19), in particular an imitation heartbeat.

8. Motor vehicle (1) according to one of the preceding claims, wherein a configuration device (21) is configured to set the audio data (20) in response to a user input and/or to select from a plurality of predetermined audio datasets (23).

## Revendications

1. Véhicule automobile (1), comportant :
- un moteur à combustion interne (2) qui produit un gaz de combustion (3) lors de son fonctionnement,
- une installation de gaz d'échappement (5) pour évacuer le gaz de combustion (5) du moteur à combustion interne (2) vers au moins un orifice de sortie (6),
- un dispositif de détection (17, 18) pour produire un signal d'état (16) qui décrit lors du fonctionnement du moteur à combustion interne (2) un bruit de moteur (4) actuel de celui-ci et/ou un paramètre de fonctionnement actuel caractérisant le fonctionnement,
- au moins un haut-parleur (11) qui est conçu pour émettre un son (13) dans un tube respectif (7) de l'installation de gaz d'échappement (3) en direction de l'au moins un orifice de sortie (6), et
- un dispositif de commande (14) qui est conçu pour commander l'au moins un haut-parleur (11) afin de produire le son (13) en fonction du signal d'état (16) et pour compenser ainsi au moins en partie le bruit de moteur (4) au moyen du son produit (13) et/ou pour le remplacer au moins en partie par un autre bruit de moteur prédéterminé,
**caractérisé en ce que**
le dispositif de commande (14) est conçu pour commander l'au moins un haut-parleur (11) en plus indépendamment du signal d'état (16) sur la base de données audio (20) prédéterminées et mémorisées et pour fournir ainsi en sortie acoustique au moyen de l'au moins un haut-parleur (11) un signal d'avertissement (19) décrit par les données audio (20), dans lequel un dispositif de détection (25) est prévu et conçu pour détecter une situation de sortie (26) prédéterminée, qui comprend au moins un changement d'état d'une installation de fermeture pour des portières de véhicule du véhicule automobile (1), et pour la signaler par un signal de déclenchement (24) indépendant du signal d'état (16) et produit dans la situation de sortie avec un retard temporel prédéterminé et dans lequel le dispositif de commande (14) est conçu pour fournir en sortie le signal d'avertissement (19) en fonction du signal de déclenchement (24).

2. Véhicule automobile (1) selon la revendication 1, dans lequel le dispositif de commande (14) est conçu pour fournir en sortie le signal d'avertissement (19) lorsque le moteur à combustion interne (2) est arrêté.

3. Véhicule automobile (1) selon l'une quelconque des revendications précédentes, dans lequel le dispositif de détection (25) est conçu pour détecter comme situation de sortie (26) une phase de stationnement dans laquelle le véhicule automobile (1) est garé et un conducteur est descendu du véhicule automobile (1).

4. Véhicule automobile (1) selon l'une quelconque des revendications précédentes, dans lequel le dispositif de détection (25) est conçu pour détecter comme situation de sortie (26) une action prédéterminée d'un utilisateur au niveau d'un dispositif de contrôle pour le véhicule automobile (1).

5. Véhicule automobile (1) selon l'une quelconque des revendications précédentes, dans lequel le dispositif de détection (25) est conçu pour détecter comme situation de sortie (26) une marche arrière passée lorsque le moteur à combustion interne (2) tourne.

6. Véhicule automobile (1) selon l'une quelconque des revendications précédentes, dans lequel le dispositif de commande (14) est conçu pour produire le signal d'avertissement (19) seulement si au moins un mode de conduite prédéterminé parmi plusieurs modes de conduite activables, par lesquels à chaque fois un autre mode de fonctionnement du moteur à combustion interne (2) et/ou d'un châssis du véhicule automobile (1) est fixé, est activé dans le véhicule automobile (1).

7. Véhicule automobile (1) selon l'une quelconque des revendications précédentes, dans lequel le dispositif de commande (14) est conçu pour fournir en sortie comme signal d'avertissement (19) un battement, en particulier un battement cardiaque imité.

8. Véhicule automobile (1) selon l'une quelconque des revendications précédentes, dans lequel un dispositif de configuration (21) est conçu pour régler les données audio (20) en fonction d'une entrée effectuée par un utilisateur et/ou pour les sélectionner parmi plusieurs jeux de données audio (23) prédéterminés.
